## Europäisches Patentamt

## European Patent Office

## Office européen des brevets

(19)

(11) Veröffentlichungsnummer: **0 101 584**
**B1**

(12) # EUROPÄISCHE PATENTSCHRIFT

(45) Veröffentlichungstag der Patentschrift:
**16.03.88**

(21) Anmeldenummer: **83107679.9**

(22) Anmeldetag: **04.08.83**

(51) Int. Cl.⁴: **B 01 J 23/74,** B 01 J 37/00,
C 07 C 85/12

(54) Geformte Eisenkatalysatormasse, deren Herstellung und Verwendung.

(30) Priorität: **21.08.82 DE 3231192**

(43) Veröffentlichungstag der Anmeldung:
**29.02.84 Patentblatt 84/9**

(45) Bekanntmachung des Hinweises auf die Patenterteilung:
**16.03.88 Patentblatt 88/11**

(84) Benannte Vertragsstaaten:
**BE DE FR GB IT NL**

(56) Entgegenhaltungen:
**EP-A-0 103 723**
**DE-A-2 034 380**
**DE-A-2 429 293**
**US-A-3 758 584**
**US-A-4 295 879**
**US-A-4 310 349**

(73) Patentinhaber: **BASF Aktiengesellschaft, Carl-Bosch- Strasse 38, D-6700 Ludwigshafen (DE)**

(72) Erfinder: **Frank, Gerhard, Dr., Heidelberger Strasse 11, D-6945 Hirschberg (DE)**
Erfinder: **Rudolf, Peter, Dr., Wiesenstrasse 1, D-6708 Neuhofen (DE)**
Erfinder: **Neubauer, Gerald, Dr., Mozartstrasse 24, D-6940 Weinheim (DE)**
Erfinder: **Ohlinger, Manfred, Dr., Anselm-Feuerbach- Strasse 13, D-6710 Frankenthal (DE)**
Erfinder: **Wilfinger, Hans Joerg, Dr., Buschstrasse 11, D-6707 Schifferstadt (DE)**
Erfinder: **Pfannebecker, Emil, Robert- Schumann-Strasse 55a, D-6707 Schifferstadt (DE)**
Erfinder: **Duffner, Paul, Dr., Bozener Strasse 14, D-6700 Ludwigshafen (DE)**

EP 0 101 584 B1

LIBER, STOCKHOLM 1988

## Beschreibung

Gegenstand der Erfindung ist eine geformte Eisenkatalysatormasse, enthaltend metallische Eisenteilchen und Gleitmittel, sowie deren Herstellung und Verwendung.

Bei der Herstellung von Aminen durch Hydrieren von Nitrilen, z. B. Hexamethylendiamin aus Adiponitril, werden Kobalt enthaltende Katalysatoren wegen ihrer hohen Selektivität bevorzugt verwendet. Solche Verfahren sind beispielsweise aus den DE-PSen 10 72 972 und 12 59 899 bekannt. Die Lebensdauer der verwendeten Kobaltkatalysatoren entspricht jedoch nicht mehr den technischen Anforderungen. Darüber hinaus weisen Befunde darauf hin, daß Stäube metallischen Kobalts und seiner schwerlöslichen Verbindungen aus arbeitshygienischen Gründen vermieden werden sollten. Es wurden auch schon Eisen enthaltende Katalysatoren für die Hydrierung von Nitrilen zu Aminen verwendet. Bei der Verwendung von Eisenkatalysatoren müssen jedoch höhere Temperaturen angewandt werden. Dies führt im verstärkten Umfang zur Bildung von Nebenprodukten wie Azacycloheptan und der schwer vom Hexamethylendiamin abtrennbaren Diamine, wie 2-Aminomethylcyclopentylamin und 1,2-Diaminocyclohexan, sowie zur Bildung von Bis-Hexamethylen-triamin und Oligomeren. So ist aus der DE-OS-24 29 293 bekannt, daß man durch Schmelzen von Magnetit und Reduktion mit Wasserstoff einen Katalysator erhält, der bei hot-spot-Temperaturen von 150 bis 170°C eine Selektivität von 98 bis 99 % an Hexamethylendiamin erzielt. Die Gehalte an 1,2-Diaminocyclohexan liegen jedoch bei 0,2 Gew.-%. Auch nach dem in der DE-AS-20 34 380 beschriebenen Verfahren, bei dem man als Katalysator eine granulierte Eisenverbindung, die durch Reduktion mit Wasserstoff in metallisches Eisen übergeführt worden ist, verwendet, erzielt man nur Selektivitäten von 97 bis 98,8 Gew.-%. Solche Eisenkatalysatoren erfüllen noch nicht alle Erwartungen, die in der Technik an sie gestellt werden und sind deshalb verbesserungsbedürftig.

Es war deshalb die technische Aufgabe gestellt, Eisenkatalysatoren zur Verfügung zu stellen, die bei der Hydrierung von Nitrilen zu Aminen eine lange Lebensdauer haben, niedrigere Hydriertemperaturen erlauben, wenig Nebenprodukte erzeugen und eine hohe Selektivität aufweisen.

Diese technische Aufgabe wird gelöst durch geformte Eisenkatalysatormassen, enthaltend metallische Eisenteilchen, die aus anisometrischen Eisenoxiden durch Kontakt mit Wasserstoff bei einer Temperatur von ≦ 500°C erhalten worden sind, und ein Gleitmittel.

Gegenstand der Erfindung ist ferner ein Verfahren zur Herstellung von geformten Eisenkatalysatormassen, wobei man anisometrische Eisenoxidteilchen bei einer Temperatur von 250 bis 500°C mit Wasserstoff zu metallischen Eisenteilchen reduziert, die so erhaltenen Eisenteilchen durch Behandeln mit einem Gemisch aus Stickstoff und Luft stabilisiert, die stabilisierten Eisenteilchen mit Gleitmitteln zu Formkörpern verpreßt und die Formkörper durch Behandeln mit Wasserstoff bei einer Temperatur von ≦ 500°C aktiviert.

Ferner ist ein Gegenstand der Erfindung die Verwendung der geformten Eisenkatalysatormassen zur Hydrierung von organischen Nitrilen zu den entsprechenden Aminen.

Die neuen Eisenkatalysatormassen haben den Vorteil, daß sie eine lange Lebensdauer haben und sich auch nach längerem Gebrauch durch überlegene mechanische Eigenschaften auszeichnen. Die neuen Katalysatoren haben ferner den Vorteil, daß sie bei niedrigeren Temperaturen eine hohe Selektivität haben. Darüber hinaus zeichnen sich die neuen Katalysatoren dadurch aus, daß weniger Nebenprodukte anfallen, die schwer von den Wertprodukten abtrennbar sind.

Die erfindungsgemäße Katalysatormasse enthält metallische Eisenteilchen, die aus anisometrischen, z. B. nadelförmigen Eisenoxidteilchen durch Kontakt mit Wasserstoff bei einer Temperatur von ≦ 500°C erhalten worden sind. Vorteilhaft weisen die metallischen Eisenteilchen einen Reduktionsgrad ≧ 95 % auf. Unter Reduktionsgrad ist der Anteil des verfügbaren Eisens in % zu verstehen, der in metallischer Form vorliegt.

Bevorzugt werden anisometrische γ-Eisenoxide, insbesondere γ-Eisen-III-oxide und γ-Eisen-III-oxidhydrat, angewandt. γ-Eisen-III-oxidhydrat, das unter dem Namen Lepidokrokit bekannt ist, wird bevorzugt verwendet. Es ist z. B. nach dem in der DE-AS 10 61 760 beschriebenen Verfahren erhältlich. Die anisometrischen Eisenoxide haben eine mittlere Teilchenlänge von 0,1 bis 2 μm, vorzugsweise 0,2 bis 1,2 μm, bei einem Längen : Dickenverhältnis von 5 : 1 bis 40 : 1 und einer spezifischen Oberfläche nach BET von 25 bis 80 m². In gleicher Weise lassen sich auch die getemperten Produkte der genannten Eisen(III)-oxide verwenden. Wobei die Temperung zweckmäßigerweise bei 250 bis 700°C erfolgt. Vorteilhaft haben die verwendeten Eisenoxide einen Alkaligehalt von kleiner 0,1 Gew.-%, berechnet als Na₂O.

Die erfindungsgemäße Eisenkatalysatormasse enthält ferner Gleitmittel, z. B. anorganische Stoffe mit Gitterstruktur, wie Talk oder Graphit. Vorteilhaft enthalten die Katalysatoren 1 bis 5 Gew.-% Gleitmittel, bezogen auf die gesamte Katalysatormasse aus Eisenteilchen und Gleitmittel. Besonders bewährt hat sich Graphit als Gleitmittel. Die erfindungsgemäße Eisenkatalysatormasse besteht somit im wesentlichen aus metallischen Eisenteilchen, die sich von nadelförmigen Eisenoxiden ableiten, geringen Mengen Eisenoxid, entsprechend dem Reduktionsgrad, und einem Gleitmittel.

Die erfindungsgemäße Eisenkatalysatormasse ist geformt, z. B. als Kugeln, Tabletten oder Strängen und weist vorteilhaft eine Druckharte von $\geq$ 300 kp/cm$^2$ auf.

Die erfindungsgemäße Katalysatormasse stellt man vorteilhaft her, indem man z. B. von γ-Eisen-III-oxiden, insbesondere γ-Eisen-III-oxidhydrat (Lepidokrokit), ausgeht. In gleicher Weise lassen sich auch die getemperten Produkte der genannten Eisen-III-oxide verwenden, wobei die Temperung zweckmäßigerweise bei 250 bis 700°C erfolgt. γ-Eisen-III-oxidhydrat erhält man beispielsweise aus wäßrigen Lösungen von Eisensalzen mit Natronlauge nach einem Verfahren, wie es in der DE-AS-10 61 760 beschrieben wird. Vorteilhaft wäscht man die γ-Eisenoxidhydratpartikel bis der Alkaligehalt kleiner 0,1 Gew.-%, berechnet als $Na_2O$, beträgt.

Die nadelförmigen Eisen-III-oxidteilchen werden mittels Wasserstoff in der Wirbelschicht, im Drehrohrofen oder vorzugsweise in einem gerührten Festbett bei einer Temperatur von 260 bis 500°C, insbesondere 300 bis 450°C innerhalb von 3 bis 36 Stunden reduziert. Vorteilhaft wendet man einen trockenen Wasserstoffstrom an, wobei man eine relativ große Wasserstoffströmungsgeschwindigkeit einhält. Es hat sich bewährt, wenn man mindestens einen 60-fachen Wasserstoffüberschuß anwendet. Vorteilhaft reduziert man solange, bis der Reduktionsgrad $\geq$ 95 % betragt. So erhaltene nadelförmige, im wesentlichen aus Eisen bestehende Metallteilchen besitzen noch weitgehend die von den Ausgangsstoffen herrührende Gestalt und sind trotz der vorangegangenen Umwandlungsreaktion einheitlich.

Anschließend werden die Metallteilchen stabilisiert. Hierunter versteht man das Umhüllen der Metallteilchen mit einer Oxidschicht durch kontrollierte Oxidation, um die durch die große freie Oberfläche der kleinen Teilchen bedingte Pyrophorität zu beseitigen. Dies wird durch Überleiten eines Luft/Stickstoff-Gemisches unter genauer Einhaltung einer vorzugsweise 100°C, insbesondere 80°C, nicht übersteigenden Temperatur über das Metallpulver erreicht. Nach der Stabilisierung soll der Reduktionsgrad nicht unter 80 %, vorzugsweise nicht unter 90 % liegen. Die stabilisierten Eisenteilchen haben eine Oberfläche nach BET von 4 bis 25 m$^2$/g, vorzugsweise 8 bis 12 m$^2$/g, sowie Partikellängen von 0,05 bis 2,0 μm sowie Porenvolumina unterhalb 0,4 cm$^3$/g, wobei das Verhältnis der Mikro- zu Makroporen zugunsten der Makroporen im Bereich von 1 : 6 bis 1 : 10 liegt. Die stabilisierten Teilchen sind anisotrop.

Die so stabilisierten Eisenteilchen werden mit einem inerten Gleitmittel, vorzugsweise Graphit, vermischt. Dabei wendet man vorteilhaft 2 bis 5 Gew.-% Gleitmittel an. Das Gemisch aus stabilisierten Eisenteilchen und Gleitmittel wird vorteilhaft unter Stickstoffabdeckung zu Formkörpern verarbeitet, z. B. zu Tabletten verpreßt. Die Druckhärte der Formkörper soll $\geq$ 300 kp/cm$^2$ betragen.

Die so erhaltenen Formkörper werden durch Behandeln mit Wasserstoff in relativ großem Überschuß, z. B. dem 60-fachen Überschuß bei einer Temperatur $\leq$ 500°C, vorzugsweise 300 bis 360°C, bei Atomosphärendruck oder unter erhöhtem Druck, z. B. 100 bis 150 bar aktiviert. Hierbei soll ein Reduktionsgrad von vorteilhaft $\geq$ 95 % erreicht werden. Durch die Aktivierung steigt die Druckhärte der Formkörper, z. B. von 300 auf 600 bis 800 kp/cm$^2$.

Die erfindungsgemäßen geformten Eisenkatalysatoren weisen eine hohe Aktivität auf, die es erlaubt, bei Hydriertemperaturen unterhalb 120°C zu arbeiten, während gemäß dem Stand der Technik bei hot-spot-Temperaturen bis zu 150° und darüber gearbeitet werden muß. Auffallend ist dabei seine geringe Neigung zur Bildung der unerwünschten cyclischen Nebenprodukte; so liegen bei der Herstellung von Hexamethylendiamin die Konzentrationen für 1,2-Diamino-cyclohexan und Azacycloheptan deutlich unter 0,2 % und für 2-Aminomethylcyclopentylamin unter 0,002 %. Der erfindungsgemäße Katalysator hat auch eine hohe mechanische Stabilität, die dadurch erreicht wird, daß man seine Verformung zu Formkörpern nicht auf der Stufe des anisometrischen Eisen-III-oxids vornimmt, sondern erst nach dessen Reduktion zu Eisenmetallteilchen und nachfolgender Stabilisierung. Geht man von Formlingen aus, die aus anisometrischem Eisen-III-oxid und Gleitmitteln hergestellt sind und anschließend reduziert worden sind, so geht die Druckhärte der Formlinge z. B. von 300 kp/cm$^2$ auf 25 kp/cm$^2$ nach Erreichen eines Reduktionsgrades von 95 % zurück. Die Katalysatorstandzeiten liegen hierbei unter 100 Tagen. Sowohl die Reduktionstemperatur als auch die Hydriertemperatur liegen höher als bei erfindungsgemäßem Verfahren und starker Selektivitätsabfall ist zu verzeichnen.

Die erfindungsgemäßen Katalysatoren eignen sich vorteilhaft zur Hydrierung von organischen Nitrilen, zu den entsprechenden Aminen.

Besonders geeignet ist der erfindungsgemäße Katalysator für die Herstellung von Alkylaminen und Alkylendiaminen durch Umsetzen von Alkannitrilen oder Alkandinitrilen mit 3 bis 18 Kohlenstoffatomen mit Wasserstoff in Gegenwart von Ammoniak. Besondere Bedeutung haben die erfindungsgemäßen Katalysatoren für die Herstellung von Hexamethylendiamin durch Umsetzen von Adipodinitril mit Wasserstoff in Gegenwart von Ammoniak. Hierbei hält man Temperaturen von 80 bis 140°C, vorzugsweise 110 bis 120°C, und Drücke von 100 bis 400 bar, vorzugsweise 200 bis 300 bar, ein. Die Hydrierung erfolgt vorteilhaft in Gegenwart von Ammoniak, das teilweise durch zurückgeführtes rohes Hydriergemisch, das im wesentlichen aus Hexamethylendiamin und Ammoniak besteht, ersetzt werden kann. Es hat sich bewährt, wenn das Volumenverhältnis von Adipodinitril zu Ammoniak wie 1 : 2 bis 1 : 20, vorzugsweise 1 : 6 bis 1 : 12 beträgt.

Der Gegenstand der Erfindung sei an folgenden Beispielen veranschaulicht.

## Beispiel 1

### Herstellung des Katalysators

600 kg nadelförmiger Lepidokrokit ($\gamma$-FeOOH) mit einem Chlorgehalt < 0,1 % und einem $Na_2$ O-Gehalt < 0,1 %, hergestellt nach der DE-AS-1 061 760, mit einer spezifischen Oberfläche von 32 $m^2$/g, einer mittleren Nadellänge von 0,8 µm und einem Längen : Dicken-Verhältnis der Nadeln von 22 : 1, einer Schüttdichte von 0,37 g/$cm^3$ und mit einem pH-Wert von 7,2 werden in einem gerührten Festbett bei 400° C, 38 h mit 400 $Nm^3$/h Wasserstoff zu metallischem Eisen (Fe $\geq$ 95 %) reduziert (stöchiometrischer Wasserstoffüberschuß : 64). Anschließend wird in einem Stickstoffluftgemisch das pyrophore nadelige Metallpigment bei einer Temperatur von 60° C mit einer stabilisierenden Oxydschicht überzogen, wobei der Reduktionsgrad nicht unter 90 % absinken soll. Die Ausbeute beträgt 400 kg. Die Sättigungsmagnetisierung der Eisenteilchen beträgt 153 nT $m^3$/g in einem 160 kA/M Meßfeld. Die Eisenteilchen haben eine spezifische Oberfläche von 7,2 $m^2$/g (nach der BET-Methode) und besitzen in der elektronenmikroskopischen Aufnahme eine anisotrope geometrische Form (Nadel bzw. Stäbchenform).

Zur Herstellung von geformten Massen mit Durchmesser 5 mm und einer Höhe von 4 mm wird das stabilisierte pulverförmige Metallpigment mit 2 Gew.-% Graphit vermischt und unter Stickstoffabdeckung tablettiert. Die Druckhärte der Tabletten soll nicht unter 300 kp/$cm^2$ liegen.

## Beispiel 2

In einem Reaktor der Länge 1 8000 mm und einem inneren Durchmesser von 160 mm werden 350 l der gemäß Beispiel 1 hergestellten Formlinge eingefüllt und bei 360° C und 150 bar mit hohem Wasserstoffüberschuß über 24 h zum Zweck der Aufhebung der Passivierung behandelt. Der Wasserstoff wird dabei im Kreis über einen Kondensator zur Abscheidung des Reduktionswassers geführt.

Nach dem Abkühlen des Katalysators wird der Reaktor in Rieselfahrweise bei 270 bar Wasserstoffdruck stündlich mit einem Gemisch von 85 l Adipinsäuredinitril und 510 l flüssigem Ammoniak unter Kreisgasfahrweise des Wasserstoffs (400 $Nm^3$/h beschickt. Die Temperatur des Zulaufgemischs beträgt 78° C und die des Reaktorausgangs liegt bei 110° C; es entstehen hot-spot-Temperaturen von max. 119° C.

Die gaschromatografische Analyse des rohen Hexamethylendiamins nach Verdampfen des Ammoniaks aus dem Hydriergemisch ergibt 0,02 Gew.-% Hexylamin, 0,09 Gew.-% Azacycloheptan, 0,11 Gew.-% 1,2-Diaminocyclohexan und 99,78 Gew.-% Hexamethylendiamin sowie einen Aminocapronitrilgehalt < 0,01 %. Der vorwiegend aus Bishexamethylentriamin bestehende Destillationsrückstand liegt bei 0,36 %. Es errechnet sich eine Selektivität von 99,4 % Hexamethylendiamin. Der Katalysator hatte nach einer Laufzeit von 400 Tagen unveränderte Aktivität und Selektivität ohne jegliche Regenerierung.

## Beispiel 3

In dem unter Beispiel 2 beschriebenen Reaktor werden an dem nach Beispiel 1 hergestellten Katalysator stündlich 70 l Adipinsäuredinitril in 430 l flüssigem Ammoniak und 490 l rückgeführtem Hydriergemisch zu Hexamethylendiamin umgesetzt. Es werden ein Druck von 250 bar Wasserstoff und 350 $Nm^3$/h Kreisgas eingehalten. Voller Umsatz des Adipinsäuredinitrils wird erreicht bei einer Zulauftemperatur von 77° C, dabei beträgt die Reaktorausgangstemperatur 104° C und Tmax. im Reaktor liegt bei 109° C.

Die gaschromatografische Analyse des rohen Hexamethylendiamins nach Verdampfen des Ammoniaks ergibt: 0,01 % Hexylamin, 0,05 % Azacycloheptan, 0,11 % 1,2 Diaminocyclohexan, 0,002 % 2-Aminomethylcyclopentylamin, 99,80 % Hexamethylendiamin und 0,01 % Aminocapronitril. Der Destillationsrückstand liegt bei 0,40 %, es errechnet sich eine Selektivität von 99,44 % Hexamethylendiamin.

## Beispiel 4

In einem Hochdruckreaktor von 2 000 mm Länge und einem inneren Durchmesser von 45 mm werden 3 l des nach Beispiel 1 hergestellten Katalysators eingefüllt und die Passivierung des Katalysators nach Beispiel 2 aufgehoben. Pro h werden dem Reaktor 100 ml Adipinsäuredinitril und 1 200 ml flüssiger Ammoniak zudosiert. Bei einer Hydriertemperatur von 109° C und einem Druck von 260 bar wird eine Selektivität von 99,63 % Hexamethylendiamin erreicht. Das rohe Hexamethylendiamin enthält nur 0,04 % Azacycloheptan und 0,09 % 1,2-Diaminocyclohexan. Der Destillationsrückstand liegt bei 0,23 %.

## Beispiel 5

In einem 2 1-Schüttelautoklaven werden 80 g 2-Methylglutarsäuredinitril und 1000 ml fl. Ammoniak in Gegenwart von 80 g Katalysatortabletten, hergestellt gemäß Beispiel 1, bei 260 bar und 100°C bis zur Beendigung der Wasserstoff-Aufnahme hydriert. Es wird bei vollständigem Umsatz des eingesetzten Dinitrils eine Selektivität von 98,8 % 2-Methylpentamethylendiamin erzielt.

Analog wird unter den o.g. Hydrierbedingungen Propionitril in fl. Ammoniak mit einer Selektivität von 97,5 % zu n-Propylamin hydriert.

## Beispiel 6

Man verfährt wie im Beispiel 4 beschrieben, verwendet jedoch 3 l Katalysator, der durch Tablettierung eines Gemisches aus 98 % γ-FeOOH und 2 % Graphit hergestellt wurde. Der Katalysator wird mittels Wasserstoff bei 450° über 72 h drucklos reduziert. Es wird ein Reduktionsgrad von 95 % erreicht. Für vollen Umsatz von 400 g Adipinsäuredinitril pro h (in 1460 g NH₃/h) ist eine Hydriertemperatur von 155°C bei 260 bar erforderlich (Rieselfahrweise).

Die Selektivität des Katalysators liegt bei 97,15 % Hexamethylendiamin. Das rohe Hexamethylendiamin enthält 1,56 % zu weit umgewandelte Produkte und 1,29 % cyclische Produkte (1,2-Diaminocyclohexan und Azacycloheptan).

## Beispiel 7

Man verfährt wie in Beispiel 4 beschrieben, verwendet jedoch 3 l eines Katalysators, der durch Reduktion von α-FeOOH und Tablettierung des hierbei entstehenden Eisenmetallpigments nach oberflächlicher Passivierung und Vermischen mit 2 % Graphit hergestellt wurde. Der Katalysator enthält durch die Fällung des α-FeOOH im alkalischen Bereich 0,18 % Natriumhydroxid eingeschlossen.

Im Reaktor wird die Passivierung durch 24stündige Behandlung mit drucklosem Wasserstoff bei 360°C aufgehoben. In Rieselfahrweise werden dann pro h 400 g Adipinsäuredinitril und 1 460 g NH₃ zudosiert.

Voller Umsatz des Adipinsäuredinitrils wird bei 172°C erzielt. Die Selektivität liegt bei 97,8 % Hexamethylendiamin, wobei 1,3 % cyclische Nebenprodukte und 0,8 % zu weit umgewandelte Produkte (vorwiegend Bishexamethylentriamin) anfallen.

## Patentansprüche

1. Geformte Eisenkatalysatormasse, enthaltend metallische Eisenteilchen, die aus anisometrischen Eisenoxidteilchen durch Kontakt mit Wasserstoff bei einer Temperatur von ≦ 500°C erhalten worden sind, und ein Gleitmittel.

2. Katalysatormassen nach Anspruch 1, dadurch gekennzeichnet, daß das verwendete Eisenoxid weniger als 0,1 Gew.-% Alkali, berechnet als Natriumoxid, enthält.

3. Katalysatormassen nach den Ansprüchen 1 und 2, dadurch gekennzeichnet, daß sie 1 bis 5 Gew.-% Gleitmittel enthalten.

4. Katalysatormassen, nach den Ansprüchen 1 bis 3, dadurch gekennzeichnet, daß sie Graphit enthalten.

5. Katalysatormassen nach den Ansprüchen 1 bis 4, dadurch gekennzeichnet, daß man von anisometrischem γ-Eisen(III)oxidhydrat ausgeht.

6. Katalysatormassen nach den Ansprüchen 1 bis 5, dadurch gekennzeichnet, daß sie eine Druckhärte von über 300 kp/cm² haben.

7. Verfahren zur Herstellung von geformten Eisenkatalysatormassen nach den Ansprüchen 1 bis 6, dadurch gekennzeichnet, daß man anisometrische γ-Eisenoxidteilchen bei einer Temperatur von 250 bis 500°C mit Wasserstoff zu metallischen Eisenteilchen reduziert, die so erhaltenen metallischen Eisenteilchen durch Behandeln mit einem Gemisch aus Stickstoff und Luft stabilisiert, die stabilisierten Eisenteilchen mit Gleitmittel zu Formkörpern verpreßt und die Formkörper durch Behandeln mit Wasserstoff bei einer Temperatur von ≦ 500°C aktiviert.

8. Verfahren nach Anspruch 7, dadurch gekennzeichnet, daß man die anisometrischen γ-Eisenoxidteilchen bis zu einem Reduktionsgrad von ≧ 95 % reduziert.

9. Verfahren nach den Ansprüchen 7 und 8, dadurch gekennzeichnet, daß man beim Stabilisieren der metallischen Eisenpigmentteilchen durch Behandeln mit einem Gemisch aus Stickstoff und Luft einen Reduktionsgrad von 80 % nicht unterschreitet.

10. Verwendung der geformten Eisenkatalysatormassen nach den Ansprüchen 1 bis 9 zur Hydrierung von organischen Nitrilen zu den entsprechenden Aminen.

## Claims

1. A molded iron catalyst which contains metallic iron particles, obtained from anisometric iron oxide particles by contact with hydrogen at ≦ 500°C, and a lubricant.

2. A catalytic material as claimed in claim 1, wherein the iron oxide used contains less than 0.1 % by weight, calculated as sodium oxide, of alkali.

3. A catalytic material as claimed in claims 1 and 2, which contains from 1 to 5 % by weight of a lubricant.

4. A catalytic material as claimed in any of claims 1 to 3, which contains graphite.

5. A catalytic material as claimed in any of claims 1 to 4, wherein the starting material used is anisometric γiron(III) oxide hydrate.

6. A catalytic material as claimed in any of claims 1 to 5, which has an indentation hardness greater than 300 kp/cm².

7. A process for the preparation of a molded iron catalyst as claimed in any of claims 1 to 6, wherein anisometric γ-iron oxide particles are reduced with hydrogen, at from 250 to 500°C, to metallic iron particles, these are stabilized by treatment with a mixture of nitrogen and air, the stabilized iron particles are pressed together with a lubricant to form moldings and the latter are activated by treatment with hydrogen at ≦ 500°C.

8. A process as claimed in claim 7, wherein the anisometric γ-iron oxide particles are reduced until the degree of reduction is ≧ 95 %.

9. A process as claimed in claims 7 and 8, wherein, when the metallic iron pigment particles are stabilized by treatment with a mixture of nitrogen and air, the degree of reduction does not fall below 80 %.

10. Use of a molded iron catalyst as claimed in any of claims 1 to 9 for the hydrogenation of organic nitriles to to the corresponding amines.

**Revendications**

1. Masse catalytique moulée, à base de fer, contenant des particules métalliques de fer, qui ont été obtenues à partir de particules d'oxyde de fer anisométriques, par contact avec de l'hydrogène, à une température de ≤ 500°C, et un lubrifiant.

2. Masses catalytiques selon la revendication 1, caractérisées par le fait que l'oxyde de fer utilisé contient moins de 0,1 % en moins d'alcali, calculé en tant qu'oxyde de sodium.

3. Masses catalytiques selon les revendications 1 et 2, caractérisés, par le fait qu'elles contiennent 1 à 5 % en poids de lubrifiant.

4. Masses catalytiques selon les revendications 1 à 3, caractérisées par le fait qu'elles contiennent du qraphite.

5. Masses catalytiques selon les revendications 1 à 4, caractérisé par le fait que l'on part d'hydrate d'oxyde de fer γ (III) anisométrique.

6. Masses catalytiques selon la revendication 1 à 5, caractérisées par le fait qu'elles ont une dureté en compression supérieure à 300 kg/cm².

7. Procédé de préparation de masses catalytiques moulées, à base de fer, selon la revendication 1 à 6, caractérisé par le fait que l'on réduit des particules d'oxyde de fer γ anisométriques, à une température de 290 à 500°C, avec de l'hydrogène, en particules de fer métalliques, on stabilise les particules de fer métalliques ainsi obtenues par traitement avec un mélange d'azote et d'air, on comprime les particules de fer stabilisées, avec un lubrifiant, en corps moulés et on active les corps moulés par traitement avec de l'hydrogène, à une température de ≤ 500°C.

8. Procédé selon la revendication 7, caractérisé par le fait que l'on réduit les particules d'oxyde de fer γ anisométrique juaqu'à un taux de réduction de ≥ 95 %.

9. Procédé selon les revendications 7 et 8 caractérisé par le fait que, lors de la stabilisation des particules de pigment de fer par traitement avec un mélange d'azote et d'air, on ne dissout pas en dessous d'un taux de réduction de 80 %.

10. Utilisation de masses catalytiques moulées à base de fer selon les revendications 1 à 9 pour l'hydrogénation de nitriles organiques en les amines correspondantes.